# FASCICULE DE BREVET EUROPEEN

(11) **EP 1 165 750 B1**
(45) Date de publication et mention de la délivrance du brevet: **09.08.2006**
(21) Numéro de dépôt: 00914252.2
(22) Date de dépôt: 24.03.2000
(51) Int. Cl.: C12N 1/00, C12R 1/01, C07K 16/12

(54) **DIAGNOSTIC DE LA MALADIE DE WHIPPLE**
DIAGNOSISVERFAHREN FÜR WHIPPLES KRANKHEIT
DIAGNOSIS OF WHIPPLE DISEASE

(30) Priorité: 26.03.1999 FR 9903989; 21.05.1999 FR 9906679
(43) Date de publication de la demande: 02.01.2002
(73) Titulaire: PROTISVALOR MEDITERRANEE, 13007 Marseille (FR)
(72) Inventeur: RAOULT, Didier, F-13008 Marseille (FR); LA SCOLA, Bernard, F-13790 Rousset (FR); BIRG, Marie-Laure, F-13400 Marseille (FR); FENOLLAR, Florence, F-13008 Marseille (FR)
(74) Mandataire: Domange, Maxime
(86) Numéro de dépôt international: PCT/FR2000/000754
(87) Numéro de publication internationale: WO 2000/058440

(56) Documents cités:
- RELMAN D.A. ET AL.: "Identification of the uncultured bacillus of Whipple's disease" NEW ENGLAND JOURNAL OF MEDICINE, vol. 327, 1992, pages 293-301, XP002123741 cité dans la demande
- DRANCOURT M.: "Tropheryma Whippelii, pathogene emergent a culture intracelulaire responsable de la maladie de Whipple" PRESSE MEDICALE, vol. 28, no. 8, 27 février 1999 (1999-02-27), pages 435-439, XP002123742
- SCHOEDON G. ET AL.: "Deactivation of macrophages with interleukin-4 is the key to the isolation of Tropheryma whippelii" JOURNAL OF INFECTIOUS DISEASES, vol. 176, no. 3, 1997, pages 672-677, XP002123743 cité dans la demande
- ZAAIJER H.L. ET AL.: "De ziekte van Whipple" NEDERLANDS TIJDSCHRIFT VOOR GENEESKUNDE, vol. 143, no. 8, 20 février 1999 (1999-02-20), pages 388-392, XP002123744
- MULLER C. ET AL.: "Cultivation of T. Whippelii from peripheral blood mononuclear cells" GASTROENTEROLOGY, vol. 116, no. 4 part 2, - avril 1999 (1999-04) page A910 XP002123745
- RAOULT D. ET AL.: "Cultivation of the bacillus of Whipple's disease" THE NEW ENGLAND JOURNAL OF MEDICINE, vol. 342, no. 9, 2 mars 2000 (2000-03-02), pages 620-625, XP000913986 cité dans la demande
- KIM B-J ET AL.: "Identification of mycobacterial species by comparative sequence analysis of the RNA polymerase gene (rpoB)" JOURNAL OF CLINICAL MICROBIOLOGY, vol. 37, no. 6, juin 1999 (1999-06), pages 1714-1720, XP000913990
- HINRIKSON H.P.: "Detection of three different types of 'Tropheryma Whippelii' directly from clinical specimens by sequencing single-strand conformation polymorphism (SSCP) analysis and type specific PCR of their 16S-23S rbosomal intergenic spacer region" INTERNATIONAL JOURNAL OF SYSTEMATIC BACTERIOLOGY, vol. 49, octobre 1999 (1999-10), pages 1701-1706, XP000914233
- MOLLET C. ET AL.: "rpoB sequence analysis as a novel basis for bacterial identification" MOLECULAR MICROBIOLOGY, vol. 26, no. 5, 1997, pages 1005-1011, XP000913977
- VON HERBAY A. ET AL.: "DIAGNOSTIC APPLICATION OF A POLYMERASE CHAIN REACTION ASSAY FOR THE WHIPPLE'S DISEASE BACTERIUM TO INTESTINAL BIOPSIES" GASTROENTEROLOGY, vol. 110, 1996, pages 1735-1743, XP000913982

## Description

La présente invention concerne le domaine du diagnostic. Plus précisément, l'invention concerne une méthode pour le diagnostic sérologique *in vitro* de la maladie de Whipple ainsi qu'un dispositif pour la mise en oeuvre de cette méthode. L'invention concerne également une trousse pour la détection *in vitro* de la bactérie responsable de la maladie Whipple.

La présente invention concerne également le domaine des techniques de détection et/ou d'amplification et séquençage à l'aide de sondes ou d'amorces oligonucléotidiques, et leur application à la recherche de la présence ou à l'identification des bactéries de l'espèce *Tropheryma whippelii.*

La maladie de Whipple est une maladie qui se présente sous des formes variées. La forme la plus classique est celle d'une fièvre avec une diarrhée chronique qui entraîne un amaigrissement, mais c'est aussi une maladie qui est susceptible de donner des atteintes articulaires chroniques, des atteintes cérébrales avec démence et aussi une atteinte cardiaque en particulier une endocardite à hémoculture négative. Dès sa description princeps en 1907, Whipple évoque l'existence d'une bactérie associée à la "lipodystrophie intestinale" devant l'observation de nombreux micro-organismes après coloration argentique d'un ganglion mésentérique (Whipple, Bull. John Hopkins Hosp. 1907 ; 18 : 328-391). La mise en évidence du caractère PAS (de l'anglais "periodic acid Schiff") positif non spécifique de cette bactérie, puis les observations en microscopie électronique confirment la présence d'une espèce bactérienne intracellulaire de structure Gram positif (Chears et al., Gastroenterology 1961 ; 41 : 129-138). L'outil moléculaire universel 16S ARNr a permis de confirmer cette hypothèse en précisant la taxonomie phylogénique de cette nouvelle espèce bactérienne, et en lui assignant le nom provisoire de *Tropheryma whipelii* pour évoquer la notion de malabsorption intestinale et honorer le découvreur de l'affection (Relman et al., N. Engl. J. Med. 1992 ; 327 : 293-301). Le séquençage direct de 721 bases d'un fragment amplifié à partir d'une biopsie de l'intestin grêle d'un patient (Wilson et al., Lancet 1991 ; 338 : 474-475), puis à partir d'un ganglion d'un autre patient (Wilson et al., ASM News 1992 ; 58 : 318-321) confirme l'originalité de l'espèce bactérienne associée à la maladie de Whipple. Le séquençage par Relman et al. (op. cité) de 1 321 bases représentant 90 % du gène sur un échantillon, et un fragment de 284 bases chez quatre autres patients a permis de confirmer que l'espèce bactérienne associée à la maladie de Whipple représentait une espèce nouvelle, de préciser sa position taxonomique dans le phylum des actinomycetes, c'est-à-dire des bactéries de structure Gram positif à haut contenu en guanosine plus cytosine, représentant un nouvel embranchement relativement proche de deux espèces connues en pathologie humaine, *Actinomyces pyogenes* et *Rothia dentocariosa.*

Le diagnostic de la maladie est actuellement fait par l'observation après coloration de frottis microscopique obtenu de biopsie ou par amplification et séquençage de l'outil du gène universel 16S ARNr (Relman et al., op. cité).

A ce jour en effet, la bactérie responsable de la maladie de Whipple n'a pas pu être isolée et cultivée de manière telle qu'une sérologie puisse être envisagée.

Contre toute attente, le Déposant a mis au point une méthode pour la culture de la bactérie responsable de la maladie de Whipple.

Les inventeurs ont découvert que la culture cellulaire qui permet l'isolement et la multiplication de la bactérie *Tropheryma whippelii* doit avoir à la fois une durée de vie longue et un temps de multiplication lent. Il ont en effet mis en évidence que le temps de dédoublement de la bactérie était très long (18 jours). De préférence, la primo-culture doit même être réalisée directement sur des cellules immortalisées.

Les travaux antérieurs réalisés sur des primo-cultures de monocytes de sang humain (SHOEDON et al. « Journal of Infectious Diseases » Volume 176, numéro 3, 1997 pages 672-677) n'ont pas permis de servir de base pour établir en culture la bactérie *Tropheryma whippelii* de façon à ce que celle-ci se multiplie, car la durée de vie moyenne de ces monocytes est de seulement 30 jours, ce qui est insuffisant compte tenu du temps de dédoublement de la bactérie.

En outre, si les cellules se multiplient trop vite, par rapport au temps de croissance de la bactérie, celles-ci ne peuvent pas être cultivées, car il se produit un effet de dilution et il devient impossible de ségréger les cellules infectées par rapport aux cellules non infectées.

Dans un mode de réalisation avantageux, les inventeurs ont utilisé des cellules de fibroblastes immortalisées. Ces cellules de fibroblastes s'étalent sur le fond de la boîte de culture, arrêtent de se multiplier quand elles ont rempli tout le tapis cellulaire, mais peuvent être maintenues plusieurs mois en vie dans ces conditions.

Plus précisément, la méthode d'isolement et culture de la bactérie qui est détaillée dans les exemples 1 et 2 ci-après, comprend l'inoculation d'un broyat de valve cardiaque sur des fibroblastes humains de la lignée HEL dans du milieu MEM. La bactérie responsable de la maladie de Whipple a été isolée et établie en culture après un minimum de deux mois d'incubation, le milieu de culture étant remplacé régulièrement. Par "établie en culture", on entend que la bactérie est obtenue de manière reproductible et se multiplie au cours du temps, notamment par repiquage successif sur culture de cellules.

La présente invention est donc relative à la bactérie ainsi isolée et établie en tant que source d'antigène. Cette bactérie a été déposée à la CNCM (Paris-France) sous le n° I-2202 et la référence d'identification TWIST- Marseille.

La présente invention a également pour objet un antigène de la bactérie selon l'invention. Plus particulièrement, la présente invention a pour objet un antigène qui est une protéine choisie parmi les protéines de poids moléculaire d'environ 10, 20, 35, 50, 60, 80, 100, 120, 150, 170 et 200 kD déterminés par électrophorèse sur gel de polyacrylamide selon la technique SDS-PAGE et par Western Blot.

La présente invention a également pour objet un anticorps spécifique dressé contre la bactérie ou un antigène selon l'invention ; plus particulièrement un anticorps polyclonal d'origine animale, notamment une immunoglobuline de souris ou de lapin, ou un anticorps monoclonal, notamment un anticorps monoclonal produit par l'hybridome déposé à la CNCM de l'Institut Pasteur sous le numéro d'enregistrement n° I-2411 et la référence d'identification TW 17G2.

La présente invention a également pour objet la détection d'un anticorps spécifique d'une immunoglobuline humaine reconnaissant ladite bactérie, de préférence IgG, IgM ou IgA, et plus particulièrement une immunoglobuline animale, notamment une immunoglobuline anti-humaine de chèvre.

La présente invention a encore plus particulièrement pour objet un antigène,
caractérisé en ce qu'il s'agit d'une protéine de 200 kD réagissant avec un anticorps monoclonal produit par les hybridomes selon l'invention ci-dessus mentionnés.

La présente invention est aussi relative à l'utilisation d'une bactérie, d'un antigène de la bactérie ou d'un anticorps spécifique selon l'invention, dans une méthode de diagnostic *in vitro* de maladies liées à des infections par la bactérie *Tropheryma whippelii,* ainsi qu'à une méthode pour le diagnostic sérologique de l'infection à bactérie *Tropheryma whippelii* selon l'invention, qui comprend la mise en contact du sérum ou de tout autre liquide biologique d'un patient avec ladite bactérie et la détection d'une réaction immunologique.

Plus particulièrement, la présente invention a pour objet une méthode de diagnostic sérologique in vitro des infections à *Tropheryma whippelii,* dans laquelle on met en contact la bactérie selon l'invention, un antigène de la bactérie selon l'invention ou un anticorps spécifique selon l'invention, avec un échantillon provenant du patient consistant dans un sérum, un fluide biologique, ou un prélèvement humain.

La méthode selon l'invention comprend l'étape consistant essentiellement à détecter une réaction immunologique entre un anticorps spécifique de la bactérie suivant l'invention et un antigène de ladite bactérie, ou entre un anticorps spécifique d'une immunoglobuline selon l'invention reconnaissant ladite bactérie et une dite immunoglobuline humaine reconnaissant ladite bactérie.

La présente invention est aussi relative à une méthode pour le diagnostic sérologique *in vitro* de la maladie de Whipple, qui comprend la mise en contact du sérum ou de tout autre liquide biologique d'un patient avec la bactérie telle que définie ci-dessus, et la détection de la réaction immunologique.

Dans un mode de réalisation, la méthode de diagnostic selon l'invention comprend :
- le dépôt dans ou sur un support solide d'une solution de bactérie selon l'invention, notamment de 0,5 à 5 µl, de préférence 1 µl de ladite solution contenant ladite bactérie ;
- l'introduction dans ou sur ledit support du sérum ou du fluide biologique à tester de préférence dilué;
- l'introduction dans ou sur le support d'une solution d'un anticorps marqué, notamment une immunoglobuline anti-humaine animale spécifique de l'immunoglobuline humaine, notamment de type IgG, IgM ou IgA, reconnaissant ladite bactérie ;
- l'observation d'une période d'incubation ;
- le rinçage éventuel du support solide et
- la détection proprement dite de la réaction immunologique entre notamment un anticorps humain reconnaissant ladite bactérie et ladite immunoglobuline anti-humaine.

Avantageusement, la méthode de diagnostic de l'invention met en oeuvre un dosage immunoenzymatique de type ELISA ou un dosage immunofluorescent. Plus particulièrement, la méthode selon l'invention comprend :
- le dépôt dans ou sur un support solide d'une solution de bactérie isolée et établie comme indiqué ci-dessus ;
- l'introduction dans ou sur ledit support du sérum ou du fluide biologique à tester dilué ;
- l'introduction dans ou sur le support d'une solution d'immunoglobuline anti-humaine marquée ;
- l'observation d'une période d'incubation ;
- le rinçage éventuel du support solide ;
- la détection proprement dite de la réaction immunologique.

Comme support solide, on peut utiliser tout dispositif adapté à la manipulation de suspensions cellulaires et bactériennes et notamment des tubes, des lames de verre, des tubes Bijoux ou des plaques rigides de microtitrage en polyéthylène, polystyrène, chlorure de polyvinyle ou nitrocellulose comportant des micropuits, les lames de verre étant préférées.

L'anticorps détecté est une immunoglobuline, notamment de type G, M ou A, spécifique de la bactérie responsable de la maladie de Whipple. Comme type de marquage de l'immunoglobubine anti-humaine, on utilise un marquage enzymatique, radioactif ou fluorescent, ce dernier type de marquage étant préféré.

L'expression "marquage fluorescent" signifie que l'anticorps a été rendu fluorescent par un agent fluorescent approprié tel que l'iso(thio)cyanate de fluorescéine combinée à une immunoglobine animale reconnaissant l'anticorps humain.

L'expression "marquage radioactif" signifie que l'anticorps porte, soit sur un élément de sa structure, par exemple les résidus de tyrosine constitutifs, soit sur un radical approprié qui lui a été fixé, un isotope radioactif permettant de le doser par comptage de la radioactivité qui lui est associée.

L'expression "marquage enzymatique" signifie que l'anticorps est couplé à une enzyme qui, associée à l'emploi de réactifs appropriés, permet une mesure quantitative de cet anticorps spécifique.

Le substrat et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant l'échantillon testé et qui fait l'objet, soit de la mesure finale spectrophotométrique ou fluorimétrique, respectivement, soit d'une évaluation à l'oeil, éventuellement par rapport à une gamme de teintes étalonnées,
- ou bien une substance colorée insoluble qui se dépose sur l'échantillon testé et qui peut faire l'objet, soit d'une mesure photométrique par réflexion, soit d'une évaluation à l'oeil, éventuellement par rapport à une gamme de teintes étalonnées.

Lorsque l'on utilise un anticorps rendu fluorescent, la fluorescence associée à l'échantillon testé est lue directement sur un appareil approprié.

Lorsque l'on utilise une sonde radioactive, comme par exemple l'iode 125, la radioactivité associée à l'échantillon testé est comptée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Lorsque l'on utilise une enzyme sur l'anticorps spécifique, l'apparition d'un produit coloré ou fluorescent est obtenue en ajoutant une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmisssion, ou en réflexion ou fluorimètre respectivement. Alternativement, on peut aussi évaluer à l'oeil la coloration obtenue, en s'aidant éventuellement d'une gamme de solutions colorées étalonnées.

En utilisant comme enzyme la phosphatase alcaline, le couplage de cette enzyme avec l'anticorps spécifique est effectué selon la méthode proposée par Boehringer Mannheim-Biochemica. Les substrats préférentiels de cette enzyme sont le paranitrophénylphosphate pour une lecture finale spectrophotométrique ou le méthyl-4-umbelliféryl phosphate pour une lecture fluorométrique ou le bromo-5 chloro-4 indolyl-3 phosphate pour obtenir un produit de réaction coloré insoluble. On peut de même utiliser comme enzyme la β-galactosidase dont les substrats préférentiels sont l'orthonitrophényl β-D-galactopyranoside ou le méthyl-4 umbelliféryl β-D-galactopyranoside.

Préférentiellement, on peut coupler les anticorps spécifiques à la peroxydase. Dans ce cas, le procédé de couplage est dérivé de celui décrit par M.B. WILSON et P.K. NAKANE in Immunofluorescence and Related Staining Techniques, W. Knapp, K. Kolubar, G. Wicks ed. Elsevier/North Holland. Amsterdam 1978, p. 215-224.

Les réactifs utilisés pour révéler la peroxydase conjuguée aux anticorps spécifiques contiennent de l'eau oxygénée, substrat de l'enzyme, et un chromogène approprié par exemple de l'orthophénylènediamine ou l'acide azino-2,2' bis(éthyl-3 thiazoline sulfonique-6) ou ABTS pour obtenir un produit final de réaction coloré et soluble dans le milieu ou bien la diamino-3,3' benzidine ou l'amino-3 éthyl-9 carbazole ou le chloro-4 α-naphtol pour obtenir un produit final de réaction insoluble, ou bien l'acide parahydroxyphényl propionique pour obtenir un produit de réaction fluorescent soluble dans le milieu.

Un autre mode de réalisation de l'invention est l'utilisation d'anticorps spécifiques couplés à l'acétylcholinestérase.

L'acétylcholinestérase est couplée à l'anticorps en utilisant préférentiellement un procédé dérivé de celui décrit dans le brevet français n° 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène reconnu par le conjugué à l'acétylcholinestérase est réalisée de préférence selon la technique bien connue qui emploie l'acétylthiocholine comme substrat de l'enzyme et le réactif d'Ellman, ou acide dithio-5,5' nitro-2 benzoïque comme chromogène, selon toute variante adaptée au cas examiné, par exemple celle décrite par Pradelles et al. dans Anal. Chem. 1985, 57: 1170-1173.

Les chromogènes cités sont utilisés tels quels ou sous forme de sels solubles dans l'eau.

La méthode de diagnostic sérologique de l'invention est adaptée à une utilisation dans des laboratoires de biologie et/ou d'anatomopathologie. Pour ce faire, on propose un dispositif pour la mise en oeuvre de cette méthode, qui comprend un support solide sur ou dans lequel on a déposé une solution contenant la bactérie telle que définie précédemment.

L'invention est aussi relative selon un autre aspect à une trousse pour la détection *in vitro* de la bactérie responsable de la maladie de Whipple. Cette trousse comprend comme composants :
- une solution contenant la bactérie ou un antigène selon l'invention, et/ou,
- une solution contenant au moins un anticorps selon l'invention et/ou,
- une solution contenant au moins un anticorps spécifique d'une immunoglobuline humaine reconnaissant ladite.

Plus particulièrement, la trousse comprend :
- une solution contenant la bactérie responsable de la maladie de Whipple, isolée et établie comme décrit ci-dessus, à titre de témoin positif;
- une solution contenant un anticorps spécifique marqué;
- éventuellement, une solution de lavage.

L'anticorps spécifique utilisé dans la trousse de l'invention est avantageusement marqué par une sonde radioactive, une enzyme ou un agent fluorescent.

Lorsque l'anticorps spécifique est marqué par une enzyme, la trousse comprend en outre le susbstrat de l'enzyme et un ou plusieurs réactifs pour visualiser l'activité de l'enzyme.

Lorsque l'anticorps spécifique est marqué par un agent fluorescent, on utilise de préférence l'iso(thio)cyanate de fluorescéine.

Selon un mode de réalisation préféré de l'invention, on utilise-comme anticorps spécifique une immunoglobuline, en particulier une immunoglobuline de souris.

La présente invention a également pour objet le gène *rpoB* de la bactérie *Tropheryma whippelii* selon la présente invention. La séquence du gène *rpoB* a été déterminée par amplification enzymatique et séquençage automatique direct avec des amorces consensus entre un grand nombre d'autres bactéries, de genres et d'espèces différents.

Le gène *rpoB* code pour une des sous-unités de l'ARN polymérase bactérienne et constitue un marqueur génétique permettant la détection spécifique de la bactérie de l'espèce *Tropheryma whippelii.*

Plus particulièrement, la présente invention a pour objet un fragment du gène *rpoB,* caractérisé en ce qu'il présente la fréquence nucléotidique SEQ ID n° 3 dans le listage des séquences en annexe.

La présente invention concerne donc également des séquences d'acides nucléiques spécifiques de l'espèce *Tiopheryma whippelii,* dont la séquence nucléotidique est tirée du gène *rpoB* de ladite bactérie et notamment du fragment du gène *rpoB* visé ci-dessus.

Selon Lazcano et al. [J. Mol. Evol. (1988) 27:365-376], les ARN polymérases sont divisées en deux groupes selon leur origine, l'un constitué par les ARN polymérases virales ARN- ou ADN-dépendantes, et l'autre constitué par les ARN polymérases ADN-dépendantes d'origine eucaryote ou procaryote (archaebactéries et eubactéries). Les ARN polymérases ADN-dépendantes eubactériennes sont
caractérisées par une constitution multimérique simple et conservée notée « core enzyme », représentée par *αββ*', ou «holoenzyme » représentée par *αββ'σ* [Yura and lshihama, Ann. Rev. Genet. (1979) 13 : 59-97].

De nombreux travaux ont mis en évidence le rôle fonctionnel, au sein du complexe enzymatique multimérique, de la sous-unité β de l'ARN polymérase eubactérienne. Les ARN polymérases archaebactérienne et eucaryote présentent, pour leur part, une structure plus complexe pouvant atteindre une dizaine, voire une trentaine de sous-unités [Pühler et al . Proc. Natl. Acad. Sci. USA (1989) 86 :4569-4573].

Les gènes qui codent pour les différentes sous-unités αββ'σ de l'ARN polymérase ADN-dépendante chez les eubactéries, respectivement les gènes *rpoA, rpoB, rpoC* et *rpoD,* sont classés en différents groupes comprenant des gènes codant pour des protéines constitutives des sous-unités ribosomiques ou pour des enzymes impliquées dans la réplication et la réparation du génome [Yura and Yshihma, Ann. Rev. Genet. (1979) 13:59-97]. Certains auteurs ont montré que les séquences nucléiques des gènes *rpoB* et *rpoC* pouvaient être utilisées afin de construire des arbres phylogénétiques [Rowland et al., Biochem. Soc. Trans. (1992) 21:40s] permettant de séparer les différents embranchements et sous-embranchements parmi les règnes du vivant.

Avant d'exposer plus en détail cet aspect de l'invention, différents termes, utilisés dans la description et les revendications, sont définis ci-après :
- par « acide nucléique extrait de bactéries » on entend soit l'acide nucléique total, soit l'ADN génomique, soit les ARN messagers, soit encore l'ADN obtenu à partir de la transcription inverse des ARN messagers ;
- un « fragment nucléotidique » ou un « oligonudéotide » sont deux termes synonymes désignant un enchaînement de motifs nucléotidiques caractérisé par une séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminées de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 10, et en particulier au moins 12 motifs nucléotidiques et peut être obtenu à partir d'une molécule d'acide nucléique naturelle et/ou par recombinaison génétique et/ou par synthèse chimique,
- un motif nucléotidique est dérivé d'un monomère qui peut être un nucléotide naturel d'acide nucléique dont les éléments constitutifs sont un sucre, un groupement phosphate et une base azotée choisie parmi l'adénine, la guanine, l'uracile, la cytosine, la thymine ; ou bien le monomère est un nucléotide modifié dans l'un au moins des trois éléments constitutifs précédents ; à titre d'exemple, la modification peut intervenir soit au niveau des bases, avec des bases modifiées telles que l'inosine, la méthyl-5-désoxycytidine, la désoxyuridine, la diméthylamino-5-désoxyuridine ou toute autre base modifiée capable d'hybridation, soit au niveau du sucre, par exemple le remplacement d'au moins un désoxyribose par un polyamide [PE Nielsen et al., Science, (1991) 254:1497-1500], soit encore au niveau du groupement phosphate, par exemple par remplacement par des esters choisis notamment parmi les diphosphates, les alkyl- et arylphosphonates et les phosporothioates,
- par « séquence informationnelle », on entend toute suite ordonnée de motifs de type nucléotidique, dont la nature chimique et l'ordre dans un sens de référence constituent une information analogue à celle donnée par la séquence des acides nucléiques naturels,
- par « hybridation », on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la « stringence », c'est-à-dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65 °C, en particulier entre 35 et 65°C dans une solution saline à une concentration d'environ 0,8 à 1 M,
- une « sonde » est un fragment nucléotidique comprenant par exemple de 10 à 100 motifs nucléotidiques, notamment de 12 à 35 motifs nucléotidiques, possédant une spécificité d'hybridation dans des conditions déterminées pour former un complexe d'hybridation avec un acide nucléique ayant, dans le cas présent, une séquence nucléotidique comprise soit dans un ARN messager, soit dans un ADN obtenu par transcription inverse dudit ARN messager, produit de transcription; une sonde peut être utilisée à des fins de diagnostic (notamment sondes de capture ou de détection) ou à des fins de thérapie,
- une « sonde de capture » est immobilisée ou immobilisable sur un support solide par tout moyen approprié, par exemple par covalence, par adsorption, ou par synthèse directe sur un solide. Des exemples de supports comprennent les plaques de microtitration et les puces à ADN,
- une « sonde de détection » peut être marquée au moyen d'un agent marqueur choisi par exemple parmi les isotopes radioactifs, les enzymes, en particulier les enzymes susceptibles d'agir sur un substrat chromogène, fluorigène ou luminescent (notamment une peroxydase ou une phosphatase alcaline), les composés chimiques chromophores, les composés chromogènes, fluorigènes ou luminescents, les analogues de bases nucléotidiques et les ligands tels que la biotine,
- une « sonde d'espèce » est une sonde permettant l'identification de l'espèce d'une bactérie,
- une « sonde de genre » est une sonde permettant l'identification du genre d'une bactérie,
- une « amorce » est une sonde comprenant par exemple de 10 à 100 motifs nucléotidiques et possédant une spécificité d'hybridation dans des conditions déterminées pour l'initiation d'une polymérisation enzymatique, par exemple dans une technique d'amplification telle que la PCR, dans un procédé de séquençage, dans une méthode de transcription, etc.

Un objet de la présente invention est un oligonucléotide monocaténaire choisi parmi les oligonucléotides ayant une séquence d'au moins 12 motifs nucléotidiques consécutifs incluse dans l'une des séquences SEQ ID N°4 et SEQ ID N°5 dans le listage des séquences en annexe et parmi les oligonucléotides complémentaires de ces oligonucléotides. Ces oligonucléotides peuvent être des oligodésoxyribonucléotides (ADN) et des oligoribonucléotides (ARN) dans lesquels « T » est remplacé par « U ».

En particulier, un oligonucléotide selon la présente invention possède au moins 12 motifs tels que décrits ci-dessus et au plus 50 motifs. Plus particulièrement, un oligonucléotide selon la présente invention possède de 12 à 35 motifs.

Un oligonucléotide préféré a une séquence choisie parmi les séquences SEQ ID N°4 et 5.

L'inosine est capable de s'apparier avec n'importe quelle autre base.

Les séquences SEQ ID N°4 et 5 peuvent être préparées par synthèse chimique en utilisant les techniques bien connues de l'homme du métier et décrites par exemple dans l'article de Itakura K. et al. [(1984) Annu. Rev. Biochem. 53:323].

Une première application d'un oligonucléotide de l'invention est son utilisation comme sonde pour la détection, dans un échantillon biologique, de bactéries de l'espèce *Tropheryma whippelii* qui comprend une séquence nucléotidique d'au moins 12 motifs nucléotidiques consécutifs incluse dans l'une des séquences SEQ ID N°4 et SEQ ID N°5, et leurs séquences complémentaires. Dans la suite de la description, une telle sonde de l'invention sera appelée sonde de l'espèce.

Les sondes selon l'invention peuvent être utilisées, à des fins de diagnostic, dans la recherche de la présence ou de l'absence d'un acide nucléique cible dans un échantillon, selon toutes les techniques d'hybridation connues et notamment les techniques de dépôt ponctuel sur filtre, dites « DOT-BLOT » [Maniatis et al. (1982) Molecular Cloning, Cold Spring Harbor], les techniques de transfert d'ADN dites « SOUTHERN BLOT » [Southern. E.M., J. Mol. Biol. (1975) 98:503], les techniques de transfert d'ARN dites « NORTHERN BLOT », ou les techniques dites « sandwich » [Dunn A.R., Hassel J.A. (1977) Cell 12:23]. On utilise en particulier la technique « sandwich », avec une sonde de capture et/ou une sonde de détection, lesdites sondes étant capables de s'hybrider avec deux régions différentes de l'acide nucléique cible, et l'une au moins desdites sondes (généralement la sonde de détection) étant capable de s'hybrider avec une région de la cible qui est spécifique de l'espèce, étant entendu que la sonde de capture et la sonde de détection doivent avoir des séquences nucléotidiques au moins partiellement différentes.

L'acide nucléique à détecter (cible) peut être de l'ADN ou de l'ARN (l'un ou l'autre éventuellement obtenu après amplification par PCR). Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80 °C.

Pour mettre en oeuvre les techniques d'hybridation précitées, et en particulier les techniques « sandwich », une sonde de l'invention, appelée sonde de capture, est immobilisée sur un support solide, et une autre sonde de l'invention. appelée sonde de détection, est marquée avec un agent marqueur.

Les exemples de support et d'agent marqueur sont tels que définis précédemment.

Un autre objet de l'invention est un procédé de détermination de la présence ou de l'absence d'une bactérie *Tropheryma whippelii,* dans un échantillon contenant ou susceptible de contenir des acides nucléiques d'au moins une telle bactérie, comprenant les étapes consistant à mettre en contact ledit échantillon avec au moins une sonde d'espèce de l'invention, puis à déterminer de façon connue en soi la formation ou l'absence de formation d'un complexe d'hybridation entre ladite sonde et l'acide nucléique de l'échantillon.

Des exemples de détection de la formation ou l'absence de formation d'uri complexe d'hybridation entre ladite sonde et l'acide nucléique comprennent les techniques décrites ci-dessus, à savoir les techniques "DOT-BLOT", "SOUTHERN-BLOT" et "sandwich".

Selon une mise en oeuvre particulière de ce procédé pour la détermination de la présence ou l'absence d'une espèce *Tropheryma whippelii,* on utilise plusieurs sondes d'espèce de l'invention, étant entendu que lesdites sondes sont capables de s'hybrider avec des régions non chevauchantes d'un acide nucléique correspondant au gène *rpoB* de *Tropheryma whippelii.*

De manière avantageuse, une sonde d'espèce est immobilisée sur un support solide, et une autre sonde d'espèce est marquée avec un agent marqueur.

Une autre application d'un oligonucléotide de l'invention est son utilisation comme amorce nucléotidique comprenant un oligonucléotide monocaténaire choisi parmi les oligonuctéotides ayant une séquence d'au moins 12 motifs nucléotidiques incluse dans l'une des séquences SEQ ID N°4 et 5, qui est utilisable dans la synthèse d'un acide nucléique en présence d'une polymérase par un procédé connu en soi, notamment dans des méthodes d'amplification utilisant une telle synthèse en présence d'une polymérase (PCR, RT-PCR, etc.). En particulier, une amorce de l'invention peut être utilisée pour la transcription inverse spécifique d'une séquence d'ARN messager de *Tropheryma whippelii* pour obtenir une séquence d'ADN complémentaire correspondante. Une telle transcription inverse peut constituer le premier stade de la technique RT-PCR, le stade suivant étant l'amplification par PCR de l'ADN complémentaire obtenu. On peut également utiliser les amorces de l'invention pour l'amplification spécifique par réaction de polymérisation en chaîne de la séquence totale de l'ADN du gène *rpoB* du *Tropheryma whippelii.*

Selon un cas particulier, ladite amorce comprenant un oligonucléotide de l'invention comprend en outre la séquence sens ou anti-sens d'un promoteur reconnu par une ARN polymérase (promoteurs T7, T3, SP6 par exemple [Studier FW, BA Moffatt (1986) J. Mol. Biol. 189:113]: de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7^{th} International Conference on AIDS (1991) Florence, Italy].

Un autre objet de l'invention est une amorce nucléotidique comprenant un oligonucléotide monocaténaire choisi parmi les oligonucléotides ayant une séquence d'au moins 12 motifs nucléotidiques consécutifs incluse dans l'une des séquences SEQ ID n°4 à SEQ ID N°5 qui est utilisable pour le séquençage total ou partiel du gène *rpoB* d'une souche quelconque de *Tropheryma whippelii.* En particulier, l'amorce nucléotidique est utilisable pour le séquençage d'un acide nucléique amplifié. Le séquençage est l'obtention de la séquence totale ou partielle du gène *ropB* par un procédé connu en soi, polymérisation absortive utilisant des di-déoxynucléotides [Sanger F., Coulson A.R. (1975) J. Mol. Biol. 94:441] ou hybridations multiples utilisant les puces à ADN.

De préférence, dans une utilisation comme amorce ou pour le séquençage des gènes *rpoB,* on utilise les séquences SEQ.ID N° 4 et 5.

Enfin, un dernier objet de l'invention est une sonde de thérapie génique pour traiter les infections provoquées par une souche de *Tropheryma whippelii,* ladite sonde comprenant un oligonucléotide tel que défini précédemment. Cette sonde de thérapie génique, capable de s'hybrider sur l'ARN messager et/ou sur l'ADN génomique desdites bactéries, peut bloquer les phénomènes de traduction et/ou de transcription et/ou de réplication.

Le principe des méthodes de thérapie génique est connu et repose notamment sur l'utilisation d'une sonde correspondant à un brin anti-sens: la formation d'un hybride entre la sonde et le brin sens est capable de perturber au moins l'une des étapes du décryptage de l'information génétique. Les sondes de thérapie génique sont donc utilisables comme médicaments antibactériens, permettant de lutter contre les infections causées par des spirochètes.

L'invention sera mieux comprise à l'aide de l'exposé ci-après, divisé en exemples, qui concernent des expériences effectuées dans le but de réaliser l'invention, et qui sont données à titre purement illustratif.

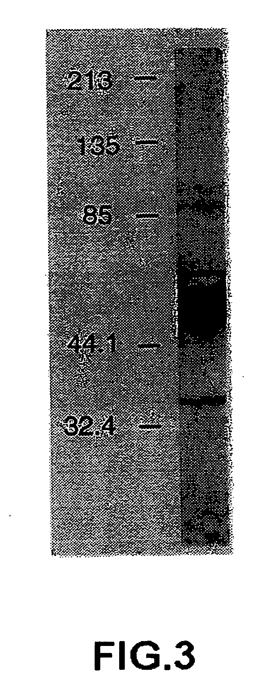

Les figures 1 à 4 sont des photographies de gel d'électrophorèse.
La figure 1 représente le profil protéique sur SDS-PAGE de *Tropheryma whippelii.*
La figure 2 représente le profil antigénique de *Tropheryma whippelii* obtenu par Western Blot.
   - Ligne 1 =: sérum de souris immunisé.
   - Ligne 2 =: sérum de lapin immunisé.
   - Ligne 3 =: sérum de patient (IgG + IgM).
   - Ligne 4 =: anticorps monoclonal 1.
   - Ligne 5 =: anticorps monoclonal 2.
   - Ligne 6 =: anticorps monoclonal 3.
   - Ligne 7 =: anticorps monoclonal 4.
La figure 3 représente un Western Blot réalisé sur la souche TWIST N° I-2202 avec un sérum de patient atteint de la maladie de Whipple avec détection d'IgM.
La figure 4 représente la visualisation du produit d'amplification du gène *rpoB* de *Tropheryma whippelii* avec des amorces SEQ ID N°1 et 2 après coloration par le bromure d'ethidium.
   - Ligne 1 :: *Tropheryma whippelii*
   - Ligne 2 :: *Nocardia otitidiscaviarum*
   - Ligne 3 :: *Mycobacterium tuberculosis*
   - Ligne 4 :: *Staphylococcus epidermidis*
   - Ligne 5 :: *Corynebacterium amycolatum*
   - Ligne 6 :: *Mycobacterium avium*
   - Ligne 7 :: *Escherichia coli*
   - Ligne 8 :: H2O
   - Ligne 9 :: H2O

### Exemple 1: Primoisolement de la bactérie

Le primoisolement a été réalisé par la technique de centrifugation sur tubes bijoux inoculés avec une lignée de fibroblastes humains HEL disponible auprès de l'ATCC. Les cellules HEL sont cultivées sur du milieu MEM (Gibco) additionné de 10 % de sérum de veau foetal (Gibco) et de 2 mM de L-glutamine (Gibco). Les tubes bijoux (Sterilin-Felthan-England, 3,7 ml) comportant une lamelle support de 12 mm de diamètre sont inoculés avec 1 ml de milieu de culture contenant environ 50 000 cellules et incubés à 37°C pendant 3 jours sous 5 % de CO₂ de façon à obtenir un tapis de cellules confluantes. La valve cardiaque étudiée a été broyée dans du milieu MEM, et la suspension a été utilisée pour inoculer les 3 tubes bijoux. Ces tubes ont ensuite été centrifugés à 700 g pendant 1 heure à 22°C. Le surnageant a ensuite été retiré, les tapis ont été lavés deux fois par du tampon PBS stérile, puis incubés avec 1 ml de milieu à 37°C sous 5 % de CO₂. Le suivi des cultures a été réalisé par cytocentrifugation de 100 µl du surnageant des tubes bijoux et coloration de Gimenez. Cette procédure a été répétée à 10, 20 et 30 jours. Au bout de 30 jours le surnageant et le tapis de cellules des tubes bijoux ont été récoltés et repiqués sur un tapis cellulaire confluent dans une boîte de culture de 25 cm² (boîte 1) avec 15 ml de milieu de culture et incubées à 37°C sous 5 % de CO₂. Chaque semaine toutes les 6 semaines suivantes (J72), le tapis cellulaire a été examiné à l'aide d'un microscope inversé à la recherche d'un effet cytopathogène et le milieu de culture a été remplacé par du milieu frais. Avant de changer le milieu, 200 µl de surnageant ont été utilisés pour réaliser une cytocentrifugation colorée par une coloration de Gimenez.

Aucun effet cytopathogène n'a été détecté avant le 65^{ème} jour. Au 72^{ème} jour, l'examen du tapis cellulaire en microscopie inverse a permis de détecter de petites inclusions sombres et irrégulières dans les cellules HEL. Sur la coloration de Gimenez de la cytocentrifugation du surnageant de la boîte 1 plusieurs bacilles fins ont été détectés, la plupart en localisation intracellulaire où ils apparaissent plus petits que les extracellulaires. Néanmoins, la plupart étaient mal ou non colorés par la coloration de Gimenez et apparaissaient bleu pâle. A la coloration de Gram de nombreux bacilles ont été aussi détectés. La plupart apparaissaient Gram positif mais plusieurs n'étaient que partiellement violet ou apparaissaient Gram négatif. A la coloration de Ziehl ces bacilles ne sont pas acido-alcoolo résistants. A la coloration par le PAS, les bacilles PAS positif apparaissaient plus nombreux que sur les colorations précédentes. La plupart des longs bacilles fins sont observables en localisation extracellulaire. Les cellules HEL apparaissent remplies de conglomérats PAS positif et de courts et fins bacilles PAS positif.

### Exemple 2 : Propagation de l'isolat

Toute la procédure de propagation a été réalisée sur des cellules HEL cultivées sur du milieu MEM additionné de 10 % de sérum de veau foetal et de 2 mM de L-glutamine et incubées à 37°C sous 5% de CO₂. Au 75^{ème} jour 3 ml de surnageant de la boîte I ont été utilisés pour inoculer 10 tubes bijoux par la méthode décrite précédemment et 2 ml de surnageant ont été utilisés pour inoculer un tapis cellulaire confluent dans une boîte de culture de 25 cm² (boîte A) avec 15 ml de milieu. Les cellules de la boîte 1 ainsi que le reste du surnageant ont été récoltés permettant d'obtenir 10 ml de suspension. Cette suspension a ensuite été divisée en 5 aliquots de 2 ml. Un des aliquots a été congelé dans de l'azote liquide. Un aliquot a été inoculé sur un tapis cellulaire confluent dans une boîte de culture de 25 cm² (boîte B) avec 15 ml de milieu. Les cellules d'un aliquot ont été lysées par 4 cycles de congélation-décongélation utilisant de l'azote liquide et de l'eau chaude (55°C) puis inoculées sur un tapis cellulaire confluent dans une boîte de culture de 25 cm² (boîte C) avec 15 ml de milieu. Un aliquot a été inoculé dans une boîte de culture saris tapis cellulaire de 25 cm² (boîte D) avec 15 ml de milieu. Au 85^{ème} jour, le milieu de toutes les boîtes et des tubes bijoux a été remplacé par du milieu frais. Les cellules ont été récoltées et inoculées dans une boîte de culture de 75 cm² (boîte D2) avec 30 ml de milieu. Avant de changer le milieu, 200 µl de surnageant ont été utilisés pour réaliser une cytocentrifugation colorée par une coloration par le PAS et le reste du surnageant a été congelé en vue d'être utilisé comme antigène pour la sérologie. Au 95^{ème} et au 105^{ème} jours, le milieu de toutes les boîtes et des tubes bijoux a été changé comme décrit précédemment. Des petites portions de tapis cellulaire ont été grattées afin de réaliser des frottis de cellules en vue d'une coloration par le PAS. L'efficacité de la propagation de la souche a été réalisée par une évaluation semi-quantitative. La présence de bacilles PAS positif a été évaluée microscopiquement en utilisant un grossissement X1000 de la façon suivante : 0, absence ; +, présents mais difficiles à trouver ; ++, faciles à trouver mais non présents dans tous les champs ; +++, présents dans tous les champs. Ces évaluations ont été réalisées à l'aveugle.

Toutes les méthodes de propagation se sont avérées efficaces puisque toutes ont permis de retrouver l'isolat après 30 jours de sous-culture (Tableau 1). L'évaluation semi-quantitative a permis d'observer que les procédures les plus efficaces sont le repiquage en tube bijoux, la sous-culture de surnageant (boîte A), et le repiquage de cellules (boîtes D, D2).

**Tableau 1**

| | | Tube bijoux | Boîte A | Boîte B | Boîte C | Boîte D | Boîte D2 |
|---|---|---|---|---|---|---|---|
| Jour 10 | Surnageant | + | - | + | - | + | NF |
| Jour 20 | Surnageant | + | - | + | + | NF | - |
| | Frottis cellulaire | NF | + | + | + | NF | - |
| Jour 30 | Surnageant | +++ | +++ | ++ | + | NF | ++ |
| | Frottis cellulaire | NF | ++ | + | + | NF | ++ |

| | | | | | | | |
|---|---|---|---|---|---|---|---|
| NF : non fait | | | | | | | |

### Exemple 3 : Détection par immunofluorescence

La détection de bactéries intracellulaires a été réalisée au 105^{ème} jour directement dans un tube bijoux par immunofluorescence. Après fixation par l'acétone, le tube a été rincé deux fois au PBS. 100 µl du sérum du patient dilué au 1:50 avec du PBS avec 3 % de lait écrémé en poudre ont été ajoutés et le tube a été incubé en chambre humide à 37°C pendant 30 mn. Après 3 rinçages au PBS le tube a été incubé pendant 30 mn à 37°C avec 100 µl d'immunoglobuline de chèvre anti-humaine marquée à l'isothiocyanate de fluorescéine (Fluoline H, Biomerieux, Marcy l'Etoile, France) diluée au 1:200 avec du PBS additionné de 0,2 % de bleu d'Evans. Après 3 rinçages au PBS la lamelle a été montée (cellules vers le bas) en glycérine tamponnée (pH 8) et examinée avec un grossissement X400 à l'aide d'un microscope à fluorescence Zeiss et d'un microscope confocal (LEICA DMIRBE) équipé d'un objectif X100 (NA. 1.4) à immersion.

L'examen en immunofluorescence montre que la coloration par le PAS ainsi que les autres colorations sous évaluent l'infection cellulaire. Sur la lamelle après 30 jours de sous-culture toutes les cellules sont remplies par l'antigène bactérien. L'étude en microscopie confocale confirme la localisation intracellulaire de la bactérie. Plusieurs bactéries sont détectées isolément sous formes de fins bacilles ressemblant à ceux observés par la coloration PAS. Néanmoins, la plupart du matériel immunopositif correspond à de plus grosses inclusions où il est impossible d'individualiser des bactéries. Il n'est pas détecté de matériel immunopositif dans le noyau des cellules.

### Exemple 4 : Microscopie électronique

Au 105^{ème} jour, 300 µl d'une solution contenant les cellules récoltées dans la boîte D2 ont été préparés pour l'étude en microscopie électronique. Les cellules ont été fixées dans une solution de glutaraldéhyde à 2,5 % en tampon cacodylate 0,1 M contenant 0.1 M de sucrose pendant 1 h à 4°C. Les cellules ont été rincées une nuit dans le même tampon puis fixées 1 h à température mbiante dans du tetraoxyde d'osmium en tamon cacodylate 0,1 M. La déshydratation a été réalisée par rinçages successifs dans des solutions d'éthanol de concentrations croissantes. Les cellules ont ensuite été incluses en blocs d'Epon 812. De fines sections ont ensuite été coupées à partir des blocs à l'aide d'un microtome LKB Ultratome III puis colorées par une solution saturée d'acétate d'uranyle dans le méthanol et une solution aqueuse de citrate de plomb avant examen sur un microscope électronique Jeol JEM 1200 EX.

L'étude en microscopie électronique confirme que les inclusions PAS positif et le matériel immunopositif correspondent à des bactéries intactes ou en voie de dégradation. La membrane cytoplasmique de ces bactéries est composée de deux couches denses aux électrons. La fine paroi bactérienne est parfois recouverte d'une pseudo-membrane externe qui donne un aspect trilamellaire. Des bactéries en cours de division sont observées.

### Exemple 5 : Production d'anticorps polyclonaux par la souris contre la bactérie responsable de la maladie Whipple

Une souris de la souche Balb C a été injectée de façon intrapéritonéale avec 0,5 ml de surnageant contenant 10⁴ bactéries responsables de la maladie de Whipple. La souris a été ensuite réinjectée 1, 2 et 3 semaines après avec 0,5 ml de la même suspension. La souris a été saignée 1 semaine après cette dernière inoculation. Le sérum a été testé d'une part contre la bactérie responsable de la maladie de Whipple en culture et d'autre part sur la valve d'un patient atteint de la maladie de Whipple, une fois par immunofluorescence et une fois par immunoperoxydase. Les anticorps révélateurs étaient des anticorps anti-souris marqués à la fluorescéine ou marqués à l'immunoperoxydase (fournis par la société Immunotech).

Les bactéries ont pu être visualisées à l'intérieur des cellules La présente demande concerne donc aussi la détection directe de la bactérie responsable de la maladie de Whipple dans les biopsies et les différents prélèvements par exemple : valve cardiaque, biopsie digestive, ou biopsie de tout autre organe suspecté d'être infecté par la bactérie responsable de la maladie de Whipple.

### Exemple 6 : Production et utilisation des anticorps monoclonaux aux produits contre Tropheryma whippelii souche Twist-Marseille et détermination d'antigènes.

### Matériel et Méthodes

Souche de *Tropheryma whippelii.* La souche de *Tropheryma whippelii* utilisée pour produire et screener les hybridomes et tester la spécificité des anticorps monoclonaux (Mabs) est la souche TWIST-Marseille déposée à la CNCM N° 1-2202. *Tropheryma whippelii* a été cultivée sur fibroblastes humains embryonnaires (HEL) dans les conditions de culture préalablement décrites. Au 75^{ème} jour, les cellules infectées d'une flasque ont été prélevées centrifugées 10 minutes à 4.000 g. Le culot de centrifugation a ensuite été remis en suspension dans 5 ml de PBS. 0.5 ml de cette suspension a été inoculé à chaque souris. Les bactéries ont également été purifiées par gradient de rénograffine et remises en suspension dans de l'eau désionisée pour le SDS-PAGE ou du PBS pour la micro-immunofluorescence (MIF).

Production d'anticorps monoclonaux (Mabs). Des souris BALB/C femelles de six semaines ont été inoculées trois fois à 7 jours d'intervalle par injection intrapéritonéale de 0.5 ml de suspension de *Tropheryma whippelii* TWIST-Marseille N° 1-2202 dans du PBS. Une semaine après la dernière des 3 injections, les souris ont reçu une dose de rappel en IV de 0.1 ml de *Tropheryma whippelii* en suspension dans du PBS. Trois jours plus tard, la rate des souris immunisées à été prélevée et les cellules spléniques ont été fusionnées avec des cellules myélomateuses SP2/0-Ag14 (10: 1) en utilisant du polyéthylène glycol à 50% (poids moléculaire : 1.300-1.600; Sigma Chemical Co., St Louis, Mo). Les cellules fusionnées ont été cultivées dans un milieu de culture pour hybridome (Seromed. Berlin, Germany) supplémenté de 20% de sérum de veau foetal (Gibco BRL) et d'hypoxanthine aminopterin-thymidine (Sigma Chemical CO., St Louis, Mo). A 37°C dans une atmosphère humide enrichie de 5% en CO2.

La présence d'anticorps anti-T. *whippelii* dans le surnageant a détectée par MIF. Les hybridomes positifs ont été sous cultivés pour la production d'ascite. Les isotypes des Mabs ont été déterminés à l'aide du kit Immuno Type Mouse Monoclonal Antibody Isotyping Kit (SIGMA) contenant des antisérums de souris IgM IgA IgG1 IgG2a IgG2b et IgG3 (Sigma). La spécificité de Mabs a été testé par western blot. Une semaine après injection intrapéritonéale aux souris de 0.5 ml de pristane (2, 62 10, 14 - tétramethypendecane ; Sigma), les anticorps en ascite ont été produits par injection intrapéritonéale de 3x10⁶ hybridomes en suspension dans 0.5 ml de PBS.

Micro-Immunofluorescence (MIF). La MIF a été utilisée pour le screening des hybridomes et pour déterminer la spécificité des Mabs. Les antigènes constitués par des cultures de *Tropheryma whippelii* ont été déposés sur des lames à 24 puits à l'aide d'une plume. Après fixation au méthanol pendant 10 minutes à température ambiante, les Mabs ont été déposés et incubés en chambre humide à 37°C pendant 30 minutes. Les lames ont ensuite été rincées deux fois 5 minutes en PBS puis dans de l'eau distillée, séchées à l'air puis incubées pendant 30 minutes à 37°C à l'aide d'anticorps de chèvre anti-IgM et anti IgG de souris conjuguées à de la fluoresceine, dilués au 1/200 dans du PBS contenant 0.2% de bleu Evans (BioMérieux, Marcy l'Etoile, France). Après rinçage, les lames ont été montées à l'aide de Fluorep (BioMérieux) puis été lues au grossissement x400 d'un microscope à fluorescence (Axioskop 20 ; Carl Zeiss, Gottinge, Germany). Les sérums des souris immunisées ont été utilisés comme contrôle positif et les sérums des souris saines comme contrôle négatif.

Pour détecter les anticorps anti- *Tropheryma whippelii* dans le sérum des patients atteints de maladie de Whipple, la MIF a été réalisée sur lames Labtech [Raoult D. et al. N. Engl. J. Med., 2000]. Les sérums ont été dilués au 1 :50, 1 :100, 1 :200, 1 :400 et 1 :800.

SDS-PAGE et Western blot. Le SDS-PAGE et le western blot ont été réalisés selon la méthode de Laemmli modifiée par l'utilisation de gel séparateur à 12% de polyacrylamide et de gel de transfert à 5%. Une suspension de *Tropheryma whippelii* contenant 4 mg de protéine/mL en tampon (0.0625 M Tris Hydrochlroride [pH8.0], 2% SDS, 5% 2-mercaptoehtanol 10% de glycérol, 0.02% de bleue de bromophénol) a été chauffée à 100°C pendant 5 minutes. Les antigènes dissous ont été séparés par électrophorèse en gel avec une intensité constante de 8 à 10 mA pendant 3 à 4 heures dans une cuve d'électrophorèse (Mini Protein II : Bio Rad, Richmond, Calif) (tampon de migration : 25 mlM Tris, 192 mM glycine, 0.1% SDS). La taille des protéines a été déterminée par comparaison à un marqueur de poids peptidique (Low Rang : Bio Rad). Les antigènes ainsi séparés ont été transférés sur une membrane de nitrocellulose (pores de 0.45 µm) à l'aide d'un tampon de transfert (2.5mM Tris, 192mM glycline, 20% methanol) sous un courant de 50 V pendant 1 heure à +4°C dans une cuve de Western Blot (Mini Trans-Blot ; Bio Rad). Après le transfert, les membranes de nitrocellulose ont été incubées toute la nuit avec une solution de 5% de lait écrémé pour bloquer les sites de fixation non spécifiques. Les membranes ont alors été lavées 3 fois en PBS puis séchées à l'air. Elles ont alors été incubées avec le surnageant des hybridomes dilués au 1 :4 ou le sérum des patients dilué au 1 :100 dans du PBS additionné de 3% de lait à température ambiante pendant 1 heure puis lavées comme décrit ci-dessus. Les membranes ont ensuite été incubées à température ambiante pendant 1 heure avec un fragment F (ab')2 d'anticorps de chèvre anti-IgG de souris conjugué à de la péroxydase (Heavy and light chains : AffiniPure ; Jackson ImmunoResearch) dilué au 1 :500 dans du PBS additionné de 3% de lait écrémé. puis lavées dans du PBS. La présence d'anticorps spécifiques a été relevée par la présence d'une activité péroxydase grâce au substrat 4chloro-1-naphtol.

Tests en aveugle des Mabs. La spécificité des anticorps monoclonaux ont été évalués en aveugle sur 19 bactéries par MIF : 12 espèces de *Bartonella: 5 B. quintana, B. henselae Marseille, B. henselae* Houston, *B. Vinsonii* Baker, B. *elizabethae, B. grahamii, B. doshiae, B. taylorii, Coxiella burnetii* Nine Mile and 6 souches variées isolées dans notre laboratoire à partir d'échantillons cliniques, dont *Listeria monocytogenes, Staphylococcus aureus, Streptococcus bovis, Mycobacterium avium, Corynebacterium* ANF group, *Actinomyces mayerii.* Après suspension dans du PBS et dépôt sur des lames à puits, la réactivité des Mabs avec les bactéries a été estimée par MIF comme décrit ci-dessus avec du liquide d'ascite dilué au 1 :100.

Patients. 15 patients ont été testés : 8 patients de maladie de Whipple à localisation digestive uniquement, dont le diagnostic avait été fait par histologie et/ou amplification de T. *whippelii* par PCR dans des biopsies digestives ; 7 patients atteints d'endocardite à *T. whippelii* par PCR dans des biopsies valvulaires.

Souris et lapins. 5 souris et 1 lapin ont été inoculée avec une suspension de T. *whippelii* afin de déterminer quels seraient les antigènes reconnus par la réponse anticorps.

### Résultats.

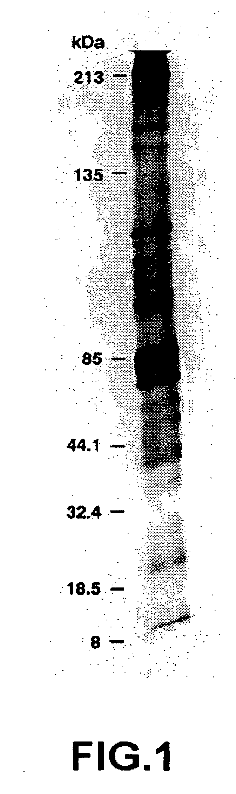

Profils de SDS-PAGE (Fig 1). Le SDS-PAGE de. T. whippelii a révélé plus particulièrement 7 bandes principales à : 10, 20, 80, 120, 150, 170, 200 kDA.

Production des Mabs. Le surnageant de 4 hybridomes a été testé par MIF. Quatre hybridomes se sont révélés spécifiques de *T*. *Whippelii.* Un hybridome a été déposé à la CNCM de l'Institut Pasteur 25 rue du Docteur Roux PARIS 75724 sous le n° I-2411 et la référence d'identification TW 17G2.

Tests sérologiques réalisés chez les patients. 13 des 15 patients testés (86.7%) ont présenté une sérologie positive, avec un taux d'IgM ≥1 :100. Le profil antigénique observé en Western Blot a révélé une réactivité avec plusieurs bandes dont une bande majeure de 200 kDA en IgG (Figure #2). En IgM plusieurs profils protéiques sont repérés dont une réactivité contre des protéines de 100, 60, 50 et 35 kDA (figure #3).

Tests sérologiques réalisés chez les souris et lapin immunisés. Les souris et le lapin immunisés ont présenté une sérologie positive, avec un taux d'lgM > 1 :100. Le profil antigénique observé en western blot a révélé une réactivité différente entre souris et lapin et différente de celle observée chez les patients. En revanche, une réactivité majeure a été observée avec une bande de 200 kDA.

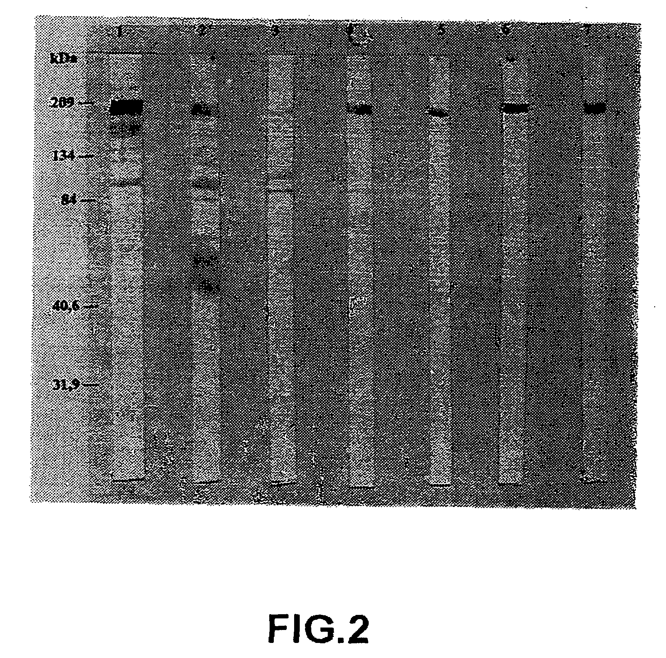

Caractérisation des Mabs (Fig 2). Les 4 Mabs ont présenté une réactivité spécifique pour un antigène de 200 kDA, déjà mis en évidence sur les western blot des patients, souris et lapin. Tous ont été identifiés comme des IgM. L'antigène reconnu a été détruit par action de la protéinase K, montrant qu'il s'agit d'une protéine.

Tests des Mabs en aveugle. L'ascite des 4 hybridomes n'a réagi qu'avec T. *whippelli.*

### Exemple 7 : Séquence du gène rpoB de Tropheryma whippelii.

La séquence du gène *rpoB* de *Tropheryma whippelii* a été déterminée par amplification enzymatique et séquençage automatique direct utilisant des amorces consensus.

Les amorces consensus utilisées avaient pour séquence
SEQ ID n° 1 = 5'-TIA TGG GII CIA AIA TGC A-3'
SEQ ID n°2 = 5'-GCC CAI CAT TCC ATI TCI CC-3' (I = Inosine)

On a incorporé de l'ADN extrait de la souche *Tropheryma whippelii* Twist-Marseille CNCM I 2202 par lyse mécanique et chimique (Fast-Prep Bio 101) dans les conditions expérimentales suivantes : 35 cycles d'amplification, chaque cycle comportant une dénaturation de l'ADN à 94°C pendant 30 sec., une hybridation initiale des amorces à 50°C pendant 30 sec. comportant un « ramping » descendant de 0,1 °C par cycle, puis une élongation à 72°C pendant 60 sec.

Les séquences SEQ ID N° 1 et SEQ ID N° 2 ont été déterminées par l'alignement des séquences peptidiques déposées dans GenBank sous les numéros d'accès suivants pour les bactéries suivantes : *Bacillus subtilis,* L43593, *Bartonella henselae,* AF171070, *Borrelia burgdorferi,* AE001144, *Buchnera aphidicola,* Z11913, *Chlamydia pneumoniae,* AE001593, *Chlamydia trachomatis,* AE001304, *Coxiella burnetti,* U86688, *Escherichia coli,* U76222, *Haemophilus influenzae,* U32733, *Helicobacter pylori,* E000625, *Legionella pneumophila,* AF087812, *Mycobacterium leprae,* Z14314, *Mycobacterium smegmatis,* U24494, *Mycobacterium tuberculosis,* L27989, *Mycoplasma gallisepticum,* L38402, *Mycoplasma genitalium,* U39715, *Mycoplasma pneumoniae,* AE000030, *Neisseria meningitidis,* Z54353, *Pseudomonas putida,* X 15849, *Rickettsia prowazekii,* AF034531, *Rickettsia typhi,* P77941, *Salmonella enterica Typhimurium,* X04642, *Spiroplasma citri,* U25815, *Staphylococcus aureus.* U970062, *Synechocystis spp.,* D90905, *Thermotoga maritima,* X72695, *Treponema pallidum,* AE001205 et *Human Granulocytic Ehrlichiosis agent,* AF237414.

Les séquences SEQ ID N° 1 et 2 ont été choisies en sélectionnant celles qui étaient les plus conservées, avec pour hypothèse qu'elles étaient aussi présentes dans *Tropheryma whippelii.*

La séquence partielle du gène *rpoB* de *Tiopheryma whippelii* (GenBank accessionnumber AF 243072) qui correspond à SEQ ID N° 3, obtenue à l'aide des amorces SEQ ID N° 1 et N° 2, est la suivante :

### Exemple 8 : Détection spécifique du gène rpoB de Tropheryma whippelii.

Des séquences spécifiques à *Tropheryma whippelii* suivantes sont été sélectionnées dans le fragment SEQ ID N° 3 :
SEQ ID N° 4 : 5'-GCA TTG TGG GGG ATG TTT-3'
SEQ ID N° 5 : 5'-TTG GGG TCA CCT TAC CAA-3'

Elles ont été choisies comme étant spécifiques à *Tropheryma whippelii* par comparaison avec les séquences connues du gène *rpoB* des 28 bactéries répertoriées dans Gen Bank mentionnées ci-dessus.

### Exemple 9: Amplification spécifique du gène rpoB de Tropheryma whippelii.

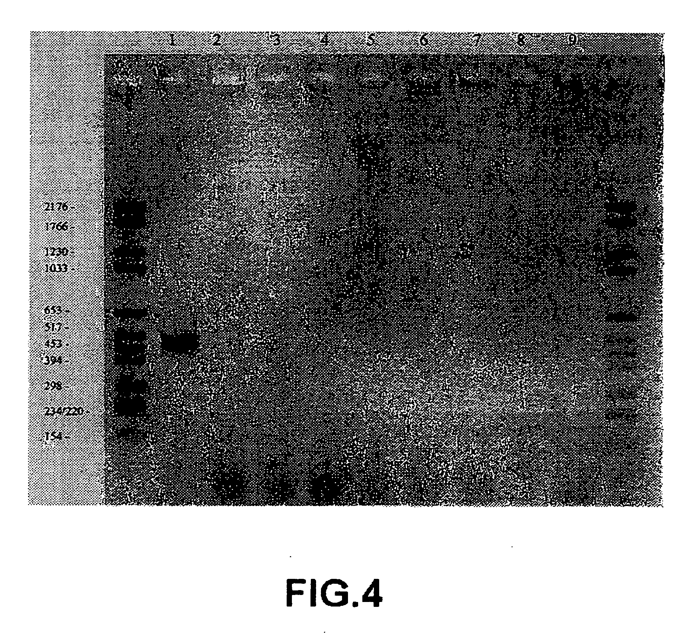

Le gène *rpoB* de *Tropheryma whippelii* a été amplifié par la technique PCR utilisant 35 cycles d'amplification comportant chacun une phase de dénaturation de 94°C pendant 30 secondes, une phase d'hybridation des amorces SEQ ID N° 4 et 5 à 52°C pendant 30 secondes et une phase d'élongation à 72°C pendant 90 secondes. Le produit d'amplification est visualisé après coloration par le bromure d'ethidium (figure 4).

Les bactéries contrôles, à savoir *Mycobacterium avium, Micobacterium Tuberculosis, Nocardia Otitidiscaviarum, Escherichia Coli, Staphylococus Eperdimilis, Corynebacterium Amycolatum,* n'ont pas été détectées, démontrant la spécificité des amorces testées.

Les séquences *rpoB* de ces espèces contrôles déposées dans GenBank sous les numéros d'accès suivants: *Mycobacterium avium* ATCC 25291, AF060336 *Mycobacterium tuberculosis* U12205, *Escherichia coli* K-12, U77436 ont été choisies du fait de leur proximité génétique avec *Tropheryma whippelii* ou du fait de leur occurrence comme contaminant possible des prélèvements cliniques soumis pour détection de *Tropheryma whippelii.*

## Revendications

1. Bactérie *Tropheryma whippelii* responsable de la maladie de Whipple isolée et établie en culture.

2. Bactérie selon la revendication 1, obtenue à partir d'une culture de cellules de fibroblaste humain après au moins 2 mois d'incubation dans un milieu de culture à base de MEM.

3. Bactérie selon la revendication 1 ou 2, **caractérisée en ce qu'**elle est déposée à la CNCM de l'Institut Pasteur sous le numéro I-2202.

4. Antigène spécifique d'une bactérie selon l'une des revendications 1 à 3.

5. Antigène selon la revendication 4, **caractérisé en ce qu'**il s'agit d'une protéine choisie parmi les protéines de poids moléculaire d'environ 100 et 200 kD déterminés sur les figures 2 et 3 par électrophorèse sur gel de polyacrylamide selon la technique Western Blot.

6. Anticorps spécifique dressé contre la bactérie ou un antigène de la bactérie selon l'une des revendications 1 à 5.

7. Anticorps suivant la revendication 6, **caractérisé en ce qu'**il s'agit d'un anticorps polyclonal d'origine animale, de préférence une immunoglobuline de souris.

8. Anticorps selon la revendication 6, **caractérisé en ce qu'**il s'agit d'un anticorps monoclonal.

9. Anticorps selon la revendication 8, **caractérisé en ce qu'**il s'agit d'un anticorps monoclonal produit par un hybridome déposé à la CNCM de l'Institut Pasteur sous le numéro d'enregistrement I-2411.

10. Antigène selon la revendication 5, **caractérisé en ce qu'**il s'agit d'une protéine de 200 kD réagissant avec un anticorps selon la revendication 9.

11. Utilisation d'une bactérie selon l'une quelconque des revendications 1 à 3 ou un antigène suivant les revendications 4, 5 ou 10 pour le diagnostic in vitro de maladies liées à des infections par la bactérie *Tropheryma whippelii.*

12. Utilisation d'un anticorps selon l'une des revendications 6 à 9, pour le diagnostic in vitro de la maladie liée à des infections par des bactéries *Tropheryma whippelii.*

13. Méthode pour le diagnostic sérologique in vitro de la maladie de Whipple qui comprend les étapes consistant essentiellement à détecter une réaction immunologique entre un anticorps spécifique de la bactérie suivant l'une des revendications 6 à 9, et un antigène de ladite bactérie selon l'une des revendications 4, 5 et 10.

14. Méthode pour le diagnostic sérologique in vitro selon la maladie de Whipple qui comprend l'étape consistant essentiellement à détecter une réaction immunologique entre un anticorps spécifique d'une immunoglobuline humaine reconnaissant ladite bactérie selon l'une des revendications 1 à 3, et une dite immunoglobuline humaine reconnaissant ladite bactérie selon l'une des revendications 1 à 3.

15. Méthode de diagnostic sérologique selon la revendication 14, qui comprend les étapes suivantes :
- le dépôt dans ou sur un support solide de solution contenant la bactérie telle qu'elle est définie dans l'une des revendications 1 à 3,
- l'introduction dans ou sur ledit support du sérum ou du fluide biologique à tester,
- l'introduction dans ou sur le support d'une solution d'un anticorps marqué spécifique d'une immunoglobuline humaine reconnaissant ladite bactérie,
- l'observation d'une période d'incubation,
- le rinçage du support solide, et
- la détection de ladite réaction immunologique.

16. Trousse pour la détection in vitro de la maladie de Whipple selon la méthode d'une des revendications 13 à 15 comprenant essentiellement comme composants :
- une solution contenant la bactérie ou un antigène tels que définis dans une des revendications 1 à 5 et 10, et/ou,
- une solution contenant au moins un anticorps selon l'une des revendications 6 à 9.

17. Trousse selon la revendication 16, **caractérisé en ce qu'**elle comprend en outre une solution contenant au moins un anticorps spécifique d'une immunoglobuline humaine reconnaissant ladite bactérie selon l'une des revendications 1 à 3.

18. Trousse suivant la revendication 16 ou 17, **caractérisée en ce qu'**elle comprend au moins un anticorps spécifique marqué.

19. Fragment du gène rpoB de la bactérie *Tropheryma whippelii* selon l'une des revendications 1 à 3, **caractérisée en ce qu'**il comprend la séquence nucléotidique SEQ ID n° 3.

20. Otigonucléotide comprenant une séquence spécifique du gène *rpoB* de la bactérie *Tropheryma whippelii* selon l'une des revendications 1 à 3, ladite séquence spécifique comprenant au moins 12 motifs nucléotidiques consécutifs inclus dans la séquence SEQ ID n° 3.

21. Oligonucléotide monocaténaire selon la revendication 20 choisi parmi les oligonucléotides ayant une séquence d'au moins 12 motifs nucléotidiques consécutifs inclue dans l'une des séquences à SEQ ID N°4 et 5, et parmi les oligonuctéotides complémentaires de ces oligonucléotides.

22. Oligonucléotide selon la revendication 20 ou 21, **caractérisé en ce qu'**il consiste dans les séquences SEQ ID N° 4 et 5.

23. Sonde pour la détection, dans un échantillon biologique, de bactéries *Tropheryma whippelii* **caractérisée en ce qu'**elle comprend une séquence selon la revendication 19 ou un oligonucléotide selon l'une des revendications 20 à 22.

24. Procédé de détermination de la présence ou de l'absence d'une bactérie *Tropheryma whippelii* dans un échantillon contenant ou susceptible de contenir des acides nucléiques d'au moins une telle bactérie, **caractérisé en ce qu'**on met en contact ledit échantillon avec au moins une sonde de la revendication 23, puis on détermine la formation ou l'absence de formation d'un complexe d'hybridation entre ladite sonde et l'acide nucléique de l'échantillon.

25. Amorce nucléotidique utilisable pour la synthèse du gène *rpoB* de *Tropheryma whippelii* en présence d'une polymérase, **caractérisée en ce qu'**elle comprend un oligonucléotide seton l'une des revendications 20 à 22, de préférence un oligonucléotide comprenant l'une des séquences SEQ ID N° 4 et SEQ ID N° 5..

## Claims

1. Bacterium *Tropheryma whippelii* responsible for Whipple's disease, isolated and established in culture.

2. Bacterium according to claim 1, obtained from a culture of human fibroblasts after at least 2 months of incubation in a culture medium based on MEM.

3. Bacterium according to claim 1 or 2, **characterised in that** it is deposited in the CNCM of the Institut Pasteur under the number I-2202.

4. Antigen of a bacterium according to one of claims 1 to 3.

5. Antigen according to claim 4, **characterised in that** it is a protein selected from those with molecular weights of about 100 and 200 kD determined in Figures 2 and 3 by polyacrylamide gel electrophoresis using the Western blot technique .

6. Specific antibody directed against the bacterium or an antigen of the bacterium according to one of claims 1 to 5.

7. Antibody according to claim 6, **characterised in that** it is a polyclonal antibody of animal origin, preferably a mouse immunoglobulin.

8. Antibody according to claim 6, **characterised in that** it is a monoclonal antibody.

9. Antibody according to claim 8, **characterised in that** it is a monoclonal antibody produced by a hybridoma deposited in the CNCM of the Institut Pasteur under the registration number I-2411.

10. Antigen according to claim 5, **characterised in that** it is a protein of 200 kD which reacts with an antibody according to claim 9.

11. Use of a bacterium according to any one of claims 1 to 3 or an antigen according to claim 4, 5 or 10 for the *in vitro* diagnosis of diseases associated with infections caused by the bacterium *Tropheryma whippelii.*

12. Use of an antibody according to one of claims 6 to 9 for *in vitro* diagnosis of the disease associated with infections caused by *Tropheryma whippelii* bacteria.

13. Method for the *in vitro* serological diagnosis of Whipple's disease, comprising the steps which consist essentially in detecting an immunological reaction between an antibody specific for the bacterium according to one of claims 6 to 9 and an antigen of said bacterium according to one of claims 4, 5 and 10.

14. Method for the *in vitro* serological diagnosis of Whipple's disease, comprising the step which consists essentially in detecting an immunological reaction between an antibody specific for a human immunoglobulin which recognizes said bacterium according to one of claims 1 to 3 and a said human immunoglobulin which recognizes said bacterium according to one of claims 1 to 3.

15. Method of serological diagnosis according to claim 14 comprising the following steps:
- depositing a solution containing the bacterium as defined in one of claims 1 to 3, in or on a solid support;
- introducing the test serum or biological fluid into or onto said support;
- introducing a solution of a labeled antibody specific for a human immunoglobulin which recognizes said bacterium, into or onto the support;
- observing an incubation period;
- rinsing the solid support; and
- detecting said immunological reaction.

16. Kit for the *in vitro* detection of Whipple's disease by the method of one of claims 13 to 15, essentially comprising the following components:
- a solution containing the bacterium or an antigen as defined in one of claims 1 to 5 and 10; and/or
- a solution containing at least one antibody according to one of claims 6 to 9.

17. Kit according to claim 16, **characterised in that** it further comprises a solution containing at least one antibody specific for a human immunoglobulin which recognizes said bacterium according to one of claims 1 to 3.

18. Kit according to claim 16 or 17, **characterised in that** it comprises at least one labeled specific antibody.

19. Fragment of the *rpoB* gene of the bacterium *Tropheryma whippelii* according to one of claims 1 to 3, **characterised in that** it has the nucleotide sequence SEQ ID N° 3.

20. Oligonucleotide comprising a sequence specific for the *rpoB* gene of the bacterium *Tropheryma whippelii* according to one of claims 1 to 3, said specific sequence comprising at least 12 consecutive nucleotide units included in the sequence SEQ ID N° 3.

21. Single-stranded oligonucleotide according to claim 20 selected from oligonucleotides having a sequence of at least 12 consecutive nucleotide units included in one of the sequences to SEQ ID N° 4 and 5, and from the oligonucleotides complementary to these oligonucleotides.

22. Oligonucleotide according to claim 20 or 21, **characterised in that** it consists of the sequences SEQ ID N° 4 and 5.

23. Probe for detecting *Tropheryma whippelii* bacteria in a biological sample, **characterised in that** it comprises a sequence according to claim 19 or an oligonucleotide according to one of claims 20 to 22.

24. Process for determining the presence or absence of a *Tropheryma whippelii* bacterium in a sample which contains or may contain nucleic acids of at least one such bacterium, **characterised in that** said sample is brought into contact with at least one probe of claim 23 and the formation or absence of formation of a hybridization complex between said probe and the nucleic acid of the sample is then determined.

25. Nucleotide primer which can be used for synthesizing the *rpoB* gene of *Tropheryma whippelii* in the presence of a polymerase, **characterised in that** it comprises an oligonucleotide according to one of claims 20 to 22, preferably an oligonucleotide comprising one of the sequences SEQ ID N° 4 and SEQ ID N° 5.

## Patentansprüche

1. Tropheryma whippelii-Bakterium, verantwortlich für die Whipple-Krankheit, isoliert oder als Kultur etabliert.

2. Bakterium nach Anspruch 1, erhalten aus einer humanen Fibroblastenzellkultur nach wenigstens zwei Monaten Inkubation in einem Kulturmedium auf Basis von MEM.

3. Bakterium nach Anspruch 1 oder 2, **dadurch gekennzeichnet, daß** es bei der CNCM des Institut Pasteur unter der Nummer I-2202 hinterlegt ist.

4. Spezifisches Antigen eines Bakteriums nach einem der Ansprüche 1 bis 3.

5. Antigen nach Anspruch 4, **dadurch gekennzeichnet, daß** es sich um ein Protein handelt, das gewählt ist unter den Proteinen mit einem Molekulargewicht von etwa 100 bis 200 kD, bestimmt in den Figuren 2 und 3 durch Elektrophorese auf Polyacrylamidgel gemäß der Western Blot Technik.

6. Spezifisch gegen das Bakterium gezogener Antikörper oder ein Antigen des Bakteriums gemäß einem der Ansprüche 1 bis 5.

7. Antikörper nach Anspruch 6, **dadurch gekennzeichnet, daß** es sich um einen polyklonalen Antikörper tierischen Ursprungs, vorzugsweise ein Mausimmunoglobulin handelt.

8. Antikörper nach Anspruch 6, **dadurch gekennzeichnet, daß** es sich um einen monoklonalen Antikörper handelt.

9. Antikörper nach Anspruch 8, **dadurch gekennzeichnet, daß** es sich um einen monoklonalen Antikörper handelt, der durch ein Hybridoma erzeugt ist, das hinterlegt ist bei der CNCM des Institut Pasteur unter der Registrierungsnummer I-2411.

10. Antigen nach Anspruch 5, **dadurch gekennzeichnet, daß** es sich um ein Protein von 200 kD handelt, das mit einem Antikörper nach Anspruch 9 reagiert.

11. Verwendung eines Bakteriums nach einem der Ansprüche 1 bis 3 oder eines Antigens nach den Ansprüchen 4, 5 oder 10 für die In-vitro-Diagnostik von Krankheiten, die verbunden sind mit Infektionen durch das Bakterium Tropheryma whippelii.

12. Verwendung eines Antikörpers nach einem der Ansprüche 6 bis 9 für die In-vitro-Diagnostik der Krankheit, die verbunden ist mit Infektionen durch Tropherymawhippelii-Bakterien.

13. Verfahren für die diagnostische In-vitro-Serologie der Whipple-Krankheit, welches die Stufen umfaßt, die im wesentlichen darin bestehen, eine immunologische Reaktion zwischen einem spezifischen Antikörper des Bakteriums gemäß einem der Ansprüche 6 bis 9 und einem Antigen des Bakteriums gemäß einem der Ansprüche 4, 5 und 10 zu detektieren.

14. Verfahren für die serologische In-vitro-Diagnostik gemäß der Whipple-Krankheit, welches die Stufe umfaßt, die im wesentlichen darin besteht, eine immunologische Reaktion zwischen einem spezifischen Antikörper eines Humanimmunoglobulins, das das Bakterium gemäß einem der Ansprüche 1 bis 3 erkennt, und einem genannten Humanimmunoglobulin, das das Bakterium gemäß einem der Ansprüche 1 bis 3 erkennt, zu detektieren.

15. Diagnostisches serologisches Verfahren nach Anspruch 14, das die folgenden Stufen umfaßt:
- die Abscheidung in oder auf einen festen Träger von einer Lösung, die das Bakterium wie definiert in einem der Ansprüche 1 bis 3 enthält,
- Einführung in oder auf den Träger des Serums oder des biologischen Fluids, das zu testen ist,
- Einführung in oder auf den Träger von einer Lösung eines spezifischen markierten Antikörpers eines Humanimmunoglobulins, das das Bakterium erkennt,
- Beobachtung eines Inkubationszeitraums,
- Spülen des festen Trägers, und
- Detektion der immunologischen Reaktion.

16. Kit für die In-vitro-Detektion der Whipple-Krankheit nach dem Verfahren von einem der Ansprüche 13 bis 15, im wesentlichen als Bestandteile umfassend:
- eine Lösung, die das Bakterium oder ein Antigen wie in einem der Ansprüche 1 bis 5 und 10 definiert enthält, und/oder
- eine Lösung, die wenigstens einen Antikörper gemäß einem der Ansprüche 6 bis 9 enthält.

17. Kit nach Anspruch 16, **dadurch gekennzeichnet, daß** er weiterhin eine Lösung enthält, die wenigstens einen spezifischen Antikörper eines Humanimmunoglobulins enthält, der das Bakterium gemäß einem der Ansprüche 1 bis 3 erkennt.

18. Kit nach einem der Ansprüche 16 oder 17, **dadurch gekennzeichnet, daß** er wenigstens einen spezifischen markierten Antikörper umfaßt.

19. Fragment des Gens rpoB des Bakteriums Tropheryma whippelii gemäß einem der Ansprüche 1 bis 3, **dadurch gekennzeichnet, daß** es die Nukleotidsequenz SEQ ID Nr. 3 umfaßt.

20. Oligonukleotid, umfassend eine spezifische Sequenz des Gens rpoB des Bakteriums Tropheryma whippelii nach einem der Ansprüche 1 bis 3, wobei die spezifische Sequenz wenigstens 12 aufeinanderfolgende Nukleotidmotive umfaßt, die in der Sequenz SEQ ID Nr. 3 eingeschlossen sind.

21. Einzelsträngiges Oligonukleotid nach Anspruch 20, gewählt unter den Oligonukleotiden mit einer Sequenz von wenigstens 12 aufeinanderfolgenden Nukleotidmotiven eingeschlossen in einer der Sequenzen mit SEQ ID Nr. 4 und 5 und unter den komplementären Oligonukleotiden dieser Oligonukleotide.

22. Oligonukleotid nach Anspruch 20 oder 21, **dadurch gekennzeichnet, daß** es aus den Sequenzen SEQ ID Nr. 4 und 5 besteht.

23. Sonde zur Detektion in einer biologischen Probe von Tropheryma whippelii-Bakterien, **dadurch gekennzeichnet, daß** sie eine Sequenz nach Anspruch 19 oder ein Oligonukleotid nach einem der Ansprüche 20 bis 22 umfaßt.

24. Verfahren zur Bestimmung der Gegenwart oder Abwesenheit eines Tropheryma whippelii-Bakteriums in einer Probe, die enthält oder geeignet ist zu enthalten Nukleinsäuren wenigstens eines solchen Bakteriums, **dadurch gekennzeichnet, daß** man die Proben mit wenigstens einer Sonde des Anspruchs 23 kontaktiert und man dann die Bildung oder Abwesenheit einer Bildung eines Hybridisierungskomplexes zwischen der Sonde und der Nukleinsäure der Probe bestimmt.

25. Nukleotidprimer, verwendbar für die Synthese des rpoB-Gens von Tropheryma whippelii in Gegenwart einer Polymerase, **dadurch gekennzeichnet, daß** er wenigstens ein Oligonukleotid nach einem der Ansprüche 20 bis 22, vorzugsweise ein Oligonukleotid umfaßt, das eine der Sequenzen SEQ ID Nr. 4 und SEQ ID Nr. 5 umfaßt.


### LISTE DE EQUENCES

<110> Université de la Méditérranée (Aix-Marseille II)
<120> Diagnostic de la maladie de Whipple
<130> H52437-2
<140>
   <141>
<150> FR 99 03989
   <151> 1999-03-26
<150> FR 99 06679
   <151> 1999-05-21
<160> 7
<170> PatentIn Ver. 2.1
<210> 1
   <211> 19
   <212> ADN
   <213> Séquence artificielle
<220>
   <223> Description de la séquence artificielle:oligonucléotide
<400> 1
   tnatgggnnc naanatgca
   19
<210> 2
   <211> 20
   <212> ADN
   <213> Séquence artificielle
<220>
   <223> Description de la séquence artificielle:oligonucléotide
<400> 2
   gcccancatt ccatntcncc
   21
<210> 3
   <211> 612
   <212> ADN
   <213> Tropheryma whippelii
<400> 3
   aagtccccng gacggnanag gnatggagng gtatgtancg atcgatgcgg gtgatgtttt
   60
   aattgccnag gatccgggca ttgtggggga tgtttccgct gatgttgtca ctgtcannca
   120
   ggatgacggg aaacatcgcg actaccatgt tggtaaattt gttcgttcaa atcagggcaa
   180
   ctgttacaac cagcnagttg tggtccgatc cggagatcgt gtataaaaag gtacagttct
   240
   tgcacatggt ccatgtactg acaaaggtga gcttagtctt ggtagaaatc ttctggttgc
   300
   tttcatgccc tgggagggct ataactttga ggatgcgata attatcagcc agaatttggt
   360
   caaggacgac accctttcnt caatccacat agaagaacat gaggttagca cccgggatac
   420
   gaagctgggc agtganagaa ataacgcgag accttccgaa tgtaagcatg gattacataa
   480
   aggacttgga cgaacggggt attatccgga ttggcgctga ggttggccct ggggacattt
   540
   tggttggtaa ggtgacccca aagggcgaag accgaactca acgcggaaga acgtttgctg
   600
   agggctatct tt
   612
<210> 4
   <211> 18
   <212> ADN
   <213> Séquence artificielle
<220>
   <223> Description de la séquence artificielle:oligonucléotide
<400> 4
   gcattgtggg ggatgttt
   18
<210> 5
   <211> 18
   <212> ADN
   <213> Séquence artificielle
<220>
   <223> Description de la séquence artificielle:oligonucléotide
<400> 5
   ttggggtcac cttaccaa
   18